# EUROPÄISCHE PATENTSCHRIFT

(11) **EP 0 448 933 B1**
(45) Veröffentlichungstag und Bekanntmachung des Hinweises auf die Patenterteilung: **10.03.1993**
(21) Anmeldenummer: 91100881.1
(22) Anmeldetag: 24.01.1991
(51) Int. Cl.: C07D 407/12

(54) **Verfahren zur Herstellung von 2,2-Dimethyl-4(2',3'-epoxy)-propoxy-methyl-1,3-dioxolan**
Process for the preparation of 2,2-dimethyl-4-(2',3'-epoxy)-propoxy-methyl-1,3-dioxolane
Procédé pour la préparation du 2,2-diméthyl-4-(2',3'-époxy)-propoxy-méthyl-1,3-dioxolanne

(30) Priorität: 27.03.1990 DE 4009739
(43) Veröffentlichungstag der Anmeldung: 02.10.1991
(73) Patentinhaber: DEUTSCHE SOLVAY-WERKE GMBH, 42697 Solingen (DE)
(72) Erfinder: Siemanowski, Werner, Dr., W-4134 Rheinberg 1 (DE); Jakobson, Gerald, Dr., W-4134 Rheinberg 2 (DE)
(74) Vertreter: Seiler, Siegfried

(56) Entgegenhaltungen:
- EP-A- 0 309 958
- DE-A- 3 220 035
- GB-A- 1 273 623
- CHEMICAL ABSTRACTS, Band 110, Nr. 10, 6. März 1989, Seite 12, Zusammenfassung Nr. 76261w, Columbus, Ohio, US; & JP-A-63 135 377 (YOKKAICHI CHEMICAL CO.) 07-06-1988

## Beschreibung

Die vorliegende Erfindung betrifft ein Verfahren zur Herstellung von 2,2-Dimethyl-4-(2′,3′-epoxy)-propoxy-methyl-1,3-dioxolan durch Umsetzung von Epichlorhydrin mit Isopropylidenglycerin unter Verwendung eines Katalysators sowie die Verwendung der Verbindung.

Aus DE-A-32 20 035 ist bereits ein Verfahren zur Herstellung von 2,2-Dimethyl-4-(2′,3′-epoxy)-propoxy-methyl-1,3-dioxolan bekannt, bei dem Acetonglycerinketal(Isopropylidenglycerin) mit Natronlauge in Gegenwart von Hexan und einem Phasentransferkatalysator (Trimethyldodecylammoniumchlorid) zusammen mit Epichlorhydrin umgesetzt wird.

Dieses Verfahren weist den Nachteil auf, daß man mit einem Lösemittel (z.B. Hexan) arbeiten, ein ca. 3 molarer Überschuß an Natronlauge (bezogen auf Acetonglycerinketal) und ein ca. zweifacher Überschuß an Epichlorhydrin eingesetzt werden muß. Es treten stark exotherme Reaktionen auf, so daß die Reaktionsführung und Steuerung schwierig ist. Durch die Mitverwendung des Lösemittels sind zusätzliche Arbeitsgänge erforderlich.

Aus GB-A-1 273 623 ist ein Verfahren zur Herstellung von Halomethylvinylglycidylethern und deren Polymerisaten bekannt. Hierbei wird zunächst ein Glycerindihalohydrin mit 0 bis 6 Molen Alkylenoxid mit mindestens 1 Mol Epihalohydrin in Gegenwart einer Lewissäure umgesetzt. Das isolierte Zwischenprodukt (Chlorhydrinether) wird mit festem Alkalimetallhydroxid oder -carbonat zum Glycidylether umgesetzt. Die Durchführung dieses Verfahrens in technischem Maßstabe ist jedoch aufwendig.

Ziel und Aufgabe der vorliegenden Erfindung war es, diese Nachteile zu vermeiden und ein technisch einfaches und wirtschaftliches Verfahren zu finden.

Die Reaktionsführung sollte vereinfacht werden und/oder unter kontrollierbaren Bedingungen ablaufen können. Das Verfahren sollte somit großtechnisch anwendbar sein. Der Einsatz organisch-chemischer Lösemittel sollte weitgehend vermieden werden. Eine Verminderung der bisher im erheblichen Überschuß, bezogen auf Isopropylidenglycerin, verwendeten Reaktanten sollte ermöglicht werden.

Erfindungsgemäß wurde festgestellt, daß diesen Zielen und Aufgaben ein Verfahren zur Herstellung von 2,2-Dimethyl-4-(2′,3′-epoxy)-propoxymethyl-1,3-dioxolan durch Umsetzung von Epichlorhydrin mit Isopropylidenglycerin unter Verwendung eines Katalysators gerecht wird, wobei Epichlorhydrin und Isopropylidenglycerin im Molverhältnis von 0,7 : 1 bis 1 : 0,7, vorzugsweise 0,8 : 1 bis 1 : 0,8, in Gegenwart mindestens einer Lewissäure, vorzugsweise BF₃-Etherat, BF₃-Essigsäure und/oder Zinntetrachlorid und/oder mindestens einer starken anorganischen Säure bei Temperaturen von 283 K bis 373 K umgesetzt, nach der Umsetzung zum Isopropylidenglycerinchlorhydrinether das nicht umgesetzte Epichlorhydrin und/oder Isopropylidenglycerin durch Destillation bei Temperaturen über 300 K, vorzugsweise bei Temperaturen von 300 bis 430 K unter Unterdruck entfernt sowie in den Kreislauf zur erneuten Umsetzung zurückgeführt, wobei vor der Destillation des nicht umgesetzten Epichlorhydrins und/oder Isopropylidenglycerins die Lewissäure(n) und/oder starke anorganische Säure(n) durch mindestens ein alkalisches Mittel neutralisiert wird und der gebildete Isopropylidenglycerinchlorhydrinether nachfolgend mit der wäßrigen Lösung mindestens einer stark alkalischen Base, vorzugsweise Natronlauge, in einem molaren Verhältnis von Chlorhydrinether zur stark alkalischen Base (berechnet ohne Wassergehalt) von 1 : 1 bis 1 : 1,1, vorzugsweise 1 : 1,005 bis 1 : 1,05, zu 2,2-Dimethyl-4-(2′,3′-epoxy) -propoxymethyl-1,3-dioxolan umgesetzt wird.

Das 2,2-Dimethyl-4-(2′,3′-epoxy)-propoxy-methyl-1,3-dioxolan kann in bekannter Weise als Zwischenprodukt zur Herstellung von nichtionogenen und/oder ionogenen Tensiden sowie als Hilfs-, Löse- und/oder Stabilisierungsmittel für organisch-chemische Verbindungen, insbesondere für chlorhaltige Verbindungen, gemäß EP-A-0 309 958, in welcher stabilisierte organische Chlorverbindungen und chlorsubstituierte Verbindungen mit C₃ oder C₄ beschrieben sind, verwendet werden.

Das 2,2-Dimethyl-4-(2′,3′-epoxy)-propxy-methyl-1,3-dioxolan ist darüber hinaus als reaktives Verdünnungsmittel, vorzugsweise für Lacke, Holzkonservierungsmittel, Farben und Anstrichmittel sowie für Kunststoffe, z.B. Plastisole und als Verarbeitungshilfsmittel für Kunststoffe geeignet.

## Patentansprüche

1. Verfahren zur Herstellung von 2,2-Dimethyl-4-(2′,3′-epoxy)-propoxy-methyl-1,3-dioxolan durch Umsetzung von Epichlorhydrin mit Isopropylidenglycerin unter Verwendung eines Katalysators, dadurch gekennzeichnet, daß Epichlorhydrin und Isopropylidenglycerin im Molverhältnis von
0,7 : 1 bis 1 : 0,7, vorzugsweise
0,8 : 1 bis 1 : 0,8,
in Gegenwart mindestens einer Lewissäure, vorzugsweise BF₃-Etherat, BF₃-Essigsäure und/oder Zinntetrachlorid, und/oder mindestens einer starken anorganischen Säure bei Temperaturen von 283 K bis 373 K umgesetzt, nach der Umsetzung zum Isopropylidenglycerinchlorhydrinether nicht umgesetztes Epichlorhydrin und/oder Isopropylidenglycerin durch Destillation bei Temperaturen über 300 K, vorzugsweise bei Temperaturen von 300 bis 430 K unter Unterdruck entfernt sowie in den Kreislauf zur erneuten Umsetzung zurückgeführt werden, wobei vor der Destillation des nicht umgesetzten Epichlorhydrins und/oder Isopropylidenglycerins die Lewissäure(n) und/oder starke anorganische(n) Säure(n) durch mindestens ein alkalisches Mittel neutralisiert wird bzw. werden, und der gebildete Isopropylidenglycerinchlorhydrinether nachfolgend mit der wäßrigen Lösung mindestens einer stark alkalischen Base, vorzugsweise Natronlauge, in einem molaren Verhältnis von Chlorhydrinether zur stark alkalischen Base (berechnet ohne Wassergehalt) von
1 : 1 bis 1 : 1,1, vorzugsweise
1 : 1,005 bis 1 : 1,05,
zu 2,2-Dimethyl-4-(2′,3′-epoxy)-propoxymethyl-1,3-dioxolan umgesetzt wird.

## Claims

1. A method for preparing 2,2-dimethyl-4-(2′,3′-epoxy)-propoxy-methyl-1,3-dioxolane by reacting epichlorohydrin with isopropylidene glycerol using a catalyst, characterised in that the epichlorohydrin and isopropylidene glycerol are reacted in a molar ratio of
0.7 : 1 to 1 : 0.7, preferably
0.8 : 1 to 1 : 0.8,
in the presence of at least one Lewis acid, preferably BF₃-etherate, BF₃-acetic acid and/or tin tetrachloride, and/or of at least one strong inorganic acid at temperatures of 283 K to 373 K, epichlorohydrin and/or isopropylidene glycerol which has not been reacted after the reaction to isopropylidene glycerol chlorohydrin ether is removed by distillation at temperatures above 300 K, preferably at temperatures of 300 to 430 K, under reduced pressure and is recycled for renewed reaction, whereby before the distillation of the non-reacted epichlorohydrin and/or isopropylidene glycerol the Lewis acid(s) and/or strong inorganic acid(s) is or are neutralised by at least one alkaline agent, and the isopropylidene glycerol chlorohydrin ether formed is then reacted with the aqueous solution of at least one strongly alkaline base, preferably sodium hydroxide solution, in a molar ratio of chlorohydrin ether to the strongly alkaline base (calculated without water content) of
1 : 1 to 1 : 1.1, preferably
1 : 1.005 to 1 : 1.05,
to form 2,2-dimethyl-4-(2′,3′-epoxy)-propoxy-methyl-1,3-dioxolane.

## Revendications

1. Procédé pour la préparation du 2,2-diméthyl-4-(2′,3′-époxy)-propoxy-méthyl-1,3-dioxolanne par la mise en réaction d'épichlorhydrine avec l'isopropylidèneglycérine en utilisant un catalyseur, caractérisé en ce qu'on fait réagir l'épichlorhydrine et l'isopropylidèneglycérine dans un rapport molaire
de 0,7 : 1 à 1 : 0,7, de préférence
de 0,8 : 1 à 1 : 0,8
en présence d'au moins un acide de Lewis, de préférence le BF₃-éthérat, le BF₃-acide acétique et/ou le tétrachlorure d'étain, et/ou au moins un acide inorganique fort à des températures de 283 K à 373 K, après la mise en réaction pour obtenir l'éther isopropylidèneglycérinechlorhydrinique, on élimine l'épichlorhydrine et/ou l'isopropylidèneglycérine qui n'ont pas réagi par distillation à des températures au-delà de 300 K, de préférence à des températures de 300 à 430 K sous pression réduite et on les remet en circulation pour une nouvelle mise en réaction, dans lequel, avant de procéder à la distillation de l'épichlorhydrine et/ou de l'isopropylidèneglycérine n'ayant pas réagi, on neutralise le ou les acides de Lewis et/ou le ou les acides inorganiques forts à l'intervention d'au moins un agent alcalin, et on fait réagir par la suite l'éther isopropylidèneglycérinechlorhydrinique obtenu avec la solution aqueuse d'au moins une base fortement alcaline, de préférence la lessive de soude dans un rapport molaire entre l'éther chlorhydrinique et la base fortement alcaline (calculé dans des conditions anhydres)
de 1 : 1 à 1 : 1,1, de préférence
de 1 : 1,005 à 1 : 1,05,
pour obtenir le 2,2-diméthyl-4-(2′,3′-époxy)-propoxy-méthyl-1,3-dioxolanne.
